(19) Europäisches Patentamt
European Patent Office
Office européen des brevets

(11) **EP 4 596 670 A1**

(12) **EUROPEAN PATENT APPLICATION**
published in accordance with Art. 153(4) EPC

(43) Date of publication:
06.08.2025  Bulletin 2025/32

(21) Application number: 23872011.4

(22) Date of filing: 15.09.2023

(51) International Patent Classification (IPC):
*C12M 3/00* (2006.01)  *C12N 5/077* (2010.01)
*C12Q 1/02* (2006.01)

(52) Cooperative Patent Classification (CPC):
C12M 3/00; C12N 5/06; C12Q 1/02

(86) International application number:
PCT/JP2023/033802

(87) International publication number:
WO 2024/070783 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.09.2022  JP 2022158753

(71) Applicant: Oji Holdings Corporation
Chuo-ku
Tokyo 104-0061 (JP)

(72) Inventors:
• SAOTOME, Hideo
Tokyo 113-8421 (JP)
• MINOWA, Osamu
Tokyo 113-8421 (JP)

• TSUCHIDA, Katsuharu
Tokyo 113-8421 (JP)
• YATSUKA, Yukiko
Tokyo 113-8421 (JP)
• OKAZAKI, Yasushi
Tokyo ,113-8421 (JP)
• TOKUNO, Hisako
Tokyo 104-0061 (JP)
• DAI, Kotaro
Tokyo 104-0061 (JP)
• SHINOTSUKA, Kei
Tokyo 104-0061 (JP)

(74) Representative: Godemeyer Blum Lenze
Patentanwälte
Partnerschaft mbB - werkpatent
An den Gärten 7
51491 Overath (DE)

(54) **CELL CULTURE SUBSTRATE EQUIPPED WITH LAYERED MYOCARDIAL CELL SHEET AND METHOD FOR MANUFACTURING SAME, AND METHOD FOR EVALUATING NORMAL MYOCARDIAL CELL SHEET FOR TRANSPLANTATION**

(57)    An object of the present invention is to provide a cell culture substrate with laminated cardiomyocyte sheets for evaluating a curative effect by biological transplantation of a normal cardiomyocyte sheet and a method for producing the same. A further object of the present invention is to provide a method for evaluating a curative effect of a normal cardiomyocyte sheet for biological transplantation. The cell culture substrate with laminated cardiomyocyte sheets of the present invention includes two or more layers of cardiomyocyte sheets on a cell culture substrate, wherein the cell culture substrate includes an alternating arrangement portion in which a strip-shaped cell adhesion region and a strip-shaped cell adhesion suppression region are alternately arranged, the cell culture substrate with laminated cardiomyocyte sheets includes the two or more layers of cardiomyocyte sheets on at least the alternating arrangement portion of the cell culture substrate; and the cell culture substrate with laminated cardiomyocyte sheets includes, as the cardiomyocyte sheets, at least one layer of each of a disease cardiomyocyte sheet containing a differentiation-induced cardiomyocyte derived from a disease patient and a normal cardiomyocyte sheet containing a differentiation-induced cardiomyocyte derived from a healthy human.

**(Cont. next page)**

[Fig. 3]

(a)

(b)

(c)

**Description**

Technical Field

The present invention relates to a cell culture substrate with laminated cardiomyocyte sheets, a method for producing the same, and a method for evaluating a normal cardiomyocyte sheet for biological transplantation.

Background Art

Treatment methods in which cardiac functions are restored by preparing a cardiomyocyte sheet in vitro and transplanting it into the affected part of the heart of a cardiac disease patient have been developed (see, for example, PTL1).

In the cardiomyocyte sheet for transplantation, there are cases of using autologous cells consisting of cells derived from a patient, obtained by preparing iPS cells from the patient's own somatic cells and inducing the cells to differentiate into cardiomyocytes, and there are other cases of using allogeneic cells derived from human different from the patient, obtained by inducing stocked healthy human-derived iPS cells to differentiate into cardiomyocytes.

Citation List

Patent Literature

PTL1: JP 2003-306434 A

Summary of Invention

Technical Problem

Whether autologous cells or allogeneic cells are used as cardiomyocyte sheets for transplantation, the preparation of cardiomyocyte sheets for transplantation requires time and high cost for preparation of transplantation such as the collection of a cell sample from a patient or healthy human, the differentiation induction from iPS cells to cardiomyocytes, and the production of cell sheets. However, it is unable to evaluate the curative effect of the transplantation with cardiomyocyte sheets until the actual transplantation treatment is performed, which has been a problem.

Thus, there is a need for a means to evaluate the transplantation effect in advance.

An object of the present invention is to provide a cell culture substrate with laminated cardiomyocyte sheets for evaluating a curative effect by biological transplantation of a normal cardiomyocyte sheet and a method for producing the same.

A further object of the present invention is to provide a method for evaluating a curative effect of a normal cardiomyocyte sheet for biological transplantation.

Solution to Problem

The objects of the present invention can be attained by configurations <1> to <7> below.

<1> A cell culture substrate with laminated cardiomyocyte sheets, including two or more layers of cardiomyocyte sheets on a cell culture substrate, wherein the cell culture substrate includes an alternating arrangement portion in which a strip-shaped cell adhesion region and a strip-shaped cell adhesion suppression region are alternately arranged, the cell culture substrate with laminated cardiomyocyte sheets includes the two or more layers of cardiomyocyte sheets on at least the alternating arrangement portion of the cell culture substrate; and the cell culture substrate with laminated cardiomyocyte sheets includes, as the cardiomyocyte sheets, at least one layer of each of a disease cardiomyocyte sheet containing a differentiation-induced cardiomyocyte derived from a disease patient and a normal cardiomyocyte sheet containing a differentiation-induced cardiomyocyte derived from a healthy human.

<2> The cell culture substrate with laminated cardiomyocyte sheets according to <1>, wherein the normal cardiomyocyte sheet is attached on the cell culture substrate and the disease cardiomyocyte sheet is laminated on the normal cardiomyocyte sheet.

<3> The cell culture substrate with laminated cardiomyocyte sheets according to <1>, wherein the disease cardiomyocyte sheet is attached on the cell culture substrate and the normal cardiomyocyte sheet is laminated on the disease cardiomyocyte sheet.

<4> A method for producing a cell culture substrate with laminated cardiomyocyte sheets, the method including in order the following steps of: preparing a cell culture substrate including an alternating arrangement portion in which a strip-shaped cell adhesion region and a strip-shaped cell adhesion suppression region are alternately arranged; forming a normal cardiomyocyte sheet containing a differentiation-induced cardiomyocyte derived from a healthy human on at least the alternating arrangement portion of the cell culture substrate; and forming a disease cardiomyocyte sheet containing a differentiation-induced cardiomyocyte derived from a disease patient on the normal cardiomyocyte sheet.

<5> A method for producing a cell culture substrate with laminated cardiomyocyte sheets, the method including in order the following steps of: preparing a cell culture substrate including an alternating arrangement portion in which a strip-shaped cell adhesion region and a strip-shaped cell adhesion suppression region are alternately arranged; forming a disease cardiomyocyte sheet containing a differentiation-induced cardiomyocyte derived from a disease patient on at least the alternating arrangement portion of the cell culture substrate; and forming a normal cardiomyocyte sheet containing a differentiation-induced cardiomyocyte derived from a healthy human on the disease cardiomyocyte sheet.

<6> A method for producing a cell culture substrate with laminated cardiomyocyte sheets, the method including the following steps of: forming a disease cardiomyocyte sheet containing a differentiation-induced cardiomyocyte derived from a disease patient on at least an alternating arrangement portion of a cell culture substrate including the alternating arrangement portion in which a strip-shaped cell adhesion region and a strip-shaped cell adhesion suppression region are alternately arranged; forming a normal cardiomyocyte sheet containing a differentiation-induced cardiomyocyte derived from a healthy human on at least an alternating arrangement portion of a cell culture substrate including the alternating arrangement portion in which a strip-shaped cell adhesion region and a strip-shaped cell adhesion suppression region are alternately arranged; and separating one of the disease cardiomyocyte sheet and the normal cardiomyocyte sheet from the cell culture substrate and laminating it on the other cardiomyocyte sheet.

<7> A method for evaluating a normal cardiomyocyte sheet for biological transplantation, the method including a step of evaluating at least one change selected from a change in motor function and a change in physiological property of the disease cardiomyocyte sheet of the cell culture substrate with laminated cardiomyocyte sheets according to any one of <1> to <3>.

Advantageous Effects of Invention

The present invention can provide a cell culture substrate with laminated cardiomyocyte sheets for evaluating a curative effect by biological transplantation of a normal cardiomyocyte sheet and a method for producing the same.
Furthermore, the present invention can provide a method for evaluating a curative effect of a normal cardiomyocyte sheet for biological transplantation.

Brief Description of Drawings

[Fig. 1] Fig. 1 is a diagram illustrating a configuration of an alternating arrangement portion in an embodiment of the alternating arrangement portion, wherein (a) is a perspective view showing the structure of the alternating arrangement portion with a petri dish, (b) is a perspective view showing a part of the surface of the alternating arrangement portion enlarged, (c) is a plan view showing a part of the surface of the alternating arrangement portion enlarged, and (d) is a partial cross-sectional view showing a part of the alternating arrangement portion enlarged.
[Fig. 2] Fig. 2 is a process diagram to illustrate an example of a method for producing an alternating arrangement portion.
[Fig. 3] Figs. 3(a) to 3(c) are schematic diagrams to illustrate production processes of a cell sheet.
[Fig. 4] Fig. 4 is a graph showing a result of a beating rate (BR) measured using a live cell imaging device.
[Fig. 5] Fig. 5 is a graph showing a result of a contractile velocity (CV) measured using a live cell imaging device.
[Fig. 6] Fig. 6 is a graph showing a result of a relaxation velocity (RV) measured using a live cell imaging device.
[Fig. 7] Fig. 7 is a graph showing a result of a contraction-relaxation duration (correction value) (CRD (correction value)) measured using a live cell imaging device.
[Fig. 8] Fig. 8 is a graph showing a result of a degree of alignment measured using a live cell imaging device.
[Fig. 9] Fig. 9 is a graph showing a line width (duration) of 50% height on day 21 of culture as measured by a calcium imaging analysis.
[Fig. 10] Fig. 10 is a graph showing duration/(interval)$^{1/2}$ calculated from a line width (duration) of 50% height and an interval of waveform peak (interval) on day 21 of culture as measured by a calcium imaging analysis.

Description of Embodiments

Hereinafter, preferred embodiments of the present invention will be described. Note that, in the present specification, "X to Y" indicating a range means "X or more and Y or less". When numerical ranges are listed stepwise, the upper and lower limits of each numerical range can be combined arbitrarily.

[Cell culture substrate with laminated cardiomyocyte sheets]

The cell culture substrate with laminated cardiomyocyte sheets of the present invention is a cell culture substrate with laminated cardiomyocyte sheets, including two or more layers of cardiomyocyte sheets on a cell culture substrate, wherein the cell culture substrate includes an alternating arrangement portion in which a strip-shaped cell adhesion region and a strip-shaped cell adhesion suppression region are alternately arranged; the cell culture substrate with laminated cardiomyocyte sheets has the two or more layers of cardiomyocyte sheets on at least the alternating arrangement portion of the cell culture substrate; and the cell culture substrate with laminated cardiomyocyte sheets has, as the cardiomyocyte sheets, at least one layer of each of a disease cardiomyocyte sheet containing a differentiation-induced cardiomyocyte derived from a disease patient and a normal cardiomyocyte sheet containing a differentiation-induced cardiomyocyte derived from a healthy human. That is, the two or more layers of cardiomyocyte sheets include at least one layer of a disease cardiomyocyte sheet containing a differentiation-induced cardiomyocyte derived from a disease patient and at least one layer of a normal cardiomyocyte sheet containing a differentiation-induced cardiomyocyte derived from a healthy human.

The cell culture substrate with laminated cardiomyocyte sheets of the present invention can be suitably used for evaluation of a normal cardiomyocyte sheet for biological transplantation.

A curative effect of a normal cardiomyocyte sheet for biological transplantation can be more accurately evaluated by using a cell culture substrate with cardiomyocyte sheets obtained by laminating a normal cardiomyocyte sheet containing a differentiation-induced cardiomyocyte derived from a healthy human (hereinafter referred to as "normal cardiomyocyte") and a disease cardiomyocyte sheet containing a differentiation-induced cardiomyocyte derived from a disease patient (hereinafter referred to as "disease cardiomyocyte") on a specific cell culture substrate as a model after biological transplantation.

Although the detailed mechanism of obtaining the above effects is unknown, a part of which can be considered as follows.

The cell culture substrate with laminated cardiomyocyte sheets of the present embodiment includes cardiomyocyte sheets on a cell culture substrate including an alternating arrangement portion in which a strip-shaped cell adhesion region and a strip-shaped cell adhesion suppression region are alternately arranged (hereinafter also referred to as "aligned culture substrate"), so that cardiomyocytes are cultured in a state in which the longitudinal directions of cardiomyocytes are aligned in one direction. In other words, the cardiomyocyte sheet becomes a cell sheet in which cardiomyocytes are aligned in one direction, so that cardiomyocytes are cultured in a state closer to in vivo. As a result, it is considered that the effect in transplanting a normal cardiomyocyte sheet can be evaluated in an environment closer to in vivo.

It should be noted that, when the disease cardiomyocytes are monolayer cultured on an aligned culture substrate, the function of the disease cardiomyocytes on the aligned culture substrate is significantly closer to the function of normal cardiomyocytes than when the disease cardiomyocytes are monolayer-cultured on a planar culture substrate. With a cardiomyocyte sheet in which the disease cardiomyocytes are aligned in one direction, the cardiomyocytes are cultured in an environment more significantly closer to in vivo and the cardiomyocyte function changes into the direction of normalization.

Thus, by analyzing a cell sheet in which the disease cardiomyocytes are monolayer-cultured on an aligned substrate, it is possible to evaluate the therapeutic effect on a disease by transplantation with normal cardiomyocytes monolayer-cultured on an aligned substrate. Furthermore, from these results, the present aligned oriented substrate having the alternating arrangement can be used as a device which shows an effect of treating diseased cardiac function for normalization by adhering directly to the affected part of diseased myocardial tissues.

As a method for maturing cardiomyocytes, culturing cells in a state close to a living body is considered effective. The myocardium in the living body is present in a unidirectional aligned state, in which electrical conduction between cells occurs via intercellular junctions, thereby performing functions such as contraction and/or relaxation.

In the present embodiment, cardiomyocyte maturation (improved physiological activity and motor function) is promoted by using an aligned culture substrate and culturing cardiomyocytes in an aligned state, and the curative effect of a normal cardiomyocyte sheet for biological transplantation is evaluated in an environment closer to in vivo.

<Cardiomyocyte>

In the present embodiment, the cardiomyocyte sheet is composed of cardiomyocytes.

Examples of the cardiomyocytes include cardiomyocytes of a vertebrate including an invertebrate, a human, and a non-human, and the vertebrate includes fish, amphibian, reptile, bird, and mammal. Specific examples of the mammal include a rodent such as a mouse, rat, ferret, hamster, guinea pig, and rabbit, a dog, a cat, a sheep, a pig, a cow, a horse, and a primate including a rhesus monkey, a chimpanzee, an orangutan, and a human. **In** addition to the mammal, the vertebrate includes fishes, poultry, and reptile.

Among them, cardiomyocytes of mammals including humans and mice are preferred, and cardiomyocytes of humans are more preferred.

In the present embodiment, the cardiomyocytes are differentiation induced cells derived from a healthy human or a disease patient.

Cardiomyocytes may be cardiomyocytes induced to differentiate from pluripotent stem cells (stem cells) by a method using a differentiation inducing factor into various mesodermal lineage differentiation-inducing. In addition, in the present embodiment, the cardiomyocytes may be induced cardiomyocytes (iCMs, cardiomyocytes obtained by direct reprogramming) induced by introducing transcription factors into fibroblasts or blood cells of a patient or the like.

Among them, the cardiomyocytes are preferably stem cell-derived cardiomyocytes.

Here, the stem cells are cells having multilineage differentiation ability and self-replication ability and examples include multipotential stem cells that can differentiate into various types of cells in a living organism such as a primate including a human and a mammal other than primate. It is preferable that the stem cells are capable of being passaged, maintain a state in which differentiation does not progress even if the stem cells are passaged, and have properties in which a karyotype or the like is hardly changed or an epigenetic phenotype is hardly changed. It is also preferable that the stem cells have sufficient proliferation capacity *in vitro* in this context. Specific examples of such a stem cell include an embryonic stem cell (hereinafter, referred to as an "ES cell"), an induced pluripotent stem cell (hereinafter, referred to as an "iPS cells"), and another artificially generated or selected stem cell having pluripotency. These stem cell may be a stem cell generated by reprogramming a somatic cell with various vectors such as retroviruses, adenoviruses, and plasmids containing specific genes, RNA, low molecular weight compounds, and the like.

Although the stem cell does not necessarily need to be a cell having pluripotency close to totipotency, but it is preferable to use a pluripotent cell having higher multipotency than a normal call. In addition, the stem cell may be a cell generated from a cell obtained from a patient with a disease, a cell that serves as a model of another disease, a cell into which a reporter gene has been incorporated (reporter cell), a cell that can be conditionally knocked out, another genetically engineered cell, or the like. This genetic engineering includes addition, modification, or deletion of a gene in a chromosome, addition of a gene or the like by various vectors or artificial chromosomes, alteration of epigenetic control, addition of an artificial genetic material such as PNA, and other genetic engineering.

Among them, the cardiomyocytes in the present embodiment are preferably cardiomyocytes derived from ES cells or iPS cells from the viewpoint of availability and differentiation induction into cardiomyocytes.

(Normal cardiomyocyte)

The normal cardiomyocyte is a differentiation induced cell derived from a healthy human, that is, a cardiomyocyte induced by differentiation from a cell collected from a healthy human. Here, the differentiation-induced normal cardiomyocyte refers to a cardiomyocyte that is not clear whether it has a specific disease and is a cardiomyocyte that constitutes a cardiomyocyte sheet for biological transplantation for a treatment of a disease.

When the cardiac disease of interest is a hereditary cardiac disease, the normal cardiomyocyte may be a normal cardiomyocyte derived from allogeneic cells or a normal cardiomyocyte that has been normalized by modifying a gene mutation by genome editing or the like.

When the cardiac disease of interest is not a hereditary cardiac disease, the normal cardiomyocyte may be a normal cardiomyocyte derived from allogeneic cells or a normal cardiomyocyte derived from autologous cells.

(Disease cardiomyocyte)

The disease cardiomyocyte is a cardiomyocyte derived from a disease patient. Here, a differentiation-induced disease cardiomyocyte is a cardiomyocyte obtained from a patient having a disease (cardiac disease), and a cardiomyocyte and a cardiomyocyte sheet derived from a disease cell have a pathology of the disease and can be used as a model.

Specific examples of the disease (cardiac disease) include cardiomyopathy (hypertrophic cardiomyopathy, dilated cardiomyopathy, constrained cardiomyopathy), heart failure (chronic heart failure, severe heart failure, congestive heart failure), ischemic cardiac disease (myocardial infarction, angina pectoris), arrhythmia, and cardiac hypertrophy due to sports.

Among these, the disease cardiomyocyte is preferably a cardiomyocyte derived from a cell established from a patient having any one disease selected from the group consisting of a hereditary cardiac disease such as hypertrophic cardiomyopathy, dilated cardiomyopathy, and constrained cardiomyopathy, and an acquired cardiac disease such as cardiac hypertrophy due to sports from the viewpoint that the disease is preferably a disease to be a target of biological transplantation in regenerative medicine.

Depending on the disease, changes in motor function such as a decrease in beating rate(BR), a decrease in contractile velocity(CV), a decrease in relaxation velocity (RV), a shortening in contraction-relaxation duration (CRD) and changes in physiological property are observed in the disease cardiomyocyte or the disease cardiomyocyte sheet compared to the normal cardiomyocyte or the normal cardiomyocyte sheet.

[Lamination of cardiomyocyte sheets]

In the present embodiment, the cell culture substrate with laminated cardiomyocyte sheets includes two or more layers of cardiomyocyte sheets on an aligned culture substrate, and includes, as the cardiomyocyte sheets, at least one layer of each of a disease cardiomyocyte sheet containing a differentiation-induced cardiomyocyte derived from a disease patient and a normal cardiomyocyte sheet containing a differentiation-induced cardiomyocyte derived from a healthy human. The disease cardiomyocyte sheet and the normal cardiomyocyte sheet are contacted on at least one side.

The laminated cardiomyocyte sheets are not particularly limited as long as it is composed of two or more layers of cardiomyocyte sheets, and may be composed of three or more layers of cardiomyocyte sheets. From the viewpoint of ease of production of cell culture substrate with laminated cardiomyocyte sheets, the number of layers of the cardiomyocyte sheet constituting the laminated cardiomyocyte sheet is preferably two layers or more and ten layers or less, more preferably two layers or more and five layers or less, further preferably two layers or more and three layers or less, and even more preferably two layers.

When the laminated cardiomyocyte sheet is composed of three or more layers of cardiomyocyte sheets, it may have a configuration in which the same type of cardiomyocyte sheets is laminated such as a disease cardiomyocyte sheet/a disease cardiomyocyte sheet/a normal cardiomyocyte sheet or a normal cardiomyocyte sheet/a normal cardiomyocyte sheet/a disease cardiomyocyte sheet, that is, a configuration in which the same type of cardiomyocyte sheets is overlaid in the lamination configuration.

Here, the laminated cardiomyocyte sheet includes, as the cardiomyocyte sheets, one or more layers of each of the normal cardiomyocyte sheet and the disease cardiomyocyte sheet.

The cell culture substrate with laminated cardiomyocyte sheets of the first embodiment has a configuration in which a normal cardiomyocyte sheet is adhered on an aligned substrate and a disease cardiomyocyte sheet is laminated on the normal cardiomyocyte sheet. With this configuration, it is possible to seed disease cardiomyocytes on an aligned normal cardiomyocyte sheet and the disease cardiomyocytes can be easily aligned, thus it is believed that it can be evaluated in an environment closer to in vivo. In addition, since the disease cardiomyocyte sheet becomes the upper layer, it is easy to observe the disease cardiomyocyte sheet.

The cell culture substrate with laminated cardiomyocyte sheets of the second embodiment has a configuration in which a disease cardiomyocyte sheet is adhered on an aligned substrate, and a normal cardiomyocyte sheet is laminated on the disease cardiomyocyte sheet. With this configuration, when the cardiomyocyte sheet is actually transplanted by regenerative medicine, it is considered that a normal cardiomyocyte sheet is transplanted onto a heart with a disease, thus it is believed that it can be evaluated in an environment closer to the actual regenerative medicine state.

[Aligned substrate]

In the present embodiment, the cell culture substrate of the cell culture substrate with laminated cardiomyocyte sheets includes an alternating arrangement portion in which a strip-shaped cell adhesion region and a strip-shaped cell adhesion suppression region are alternately arranged.

The cell adhesion region is a flat portion, and the cell adhesion suppression region is a recession and protrusion portion including a plurality of recessions and protrusions. Each of the flat portions has a shape extending in a first direction, and a plurality of the flat portions are arrayed in a second direction intersecting the first direction over an entirety of the surface. Each of the recession and protrusion portions includes a plurality of stepped structures filling a gap between the flat portions adjacent to each other. In other words, the cell culture substrate includes an alternating arrangement portion in which a strip-shaped flat portion, which is a cell adhesion region, and a recession and protrusion portion, which is a cell adhesion suppression region, are alternately arranged.

The pitch of the stepped structure of the recession and protrusion portion is preferably 100 nm or more and 10 $\mu$m or less. The stepped structures each include a protrusion portion, the recession and protrusion portions each include a plurality of the protrusion portions in a bottom surface of a recession portion sandwiched between the flat portions adjacent to each other, and a difference between a height of a distal end surface of each of the recession and protrusion portions and a height of each of the flat portions is preferably 0.5 $\mu$m or less in a thickness direction of the alternating arrangement portion.

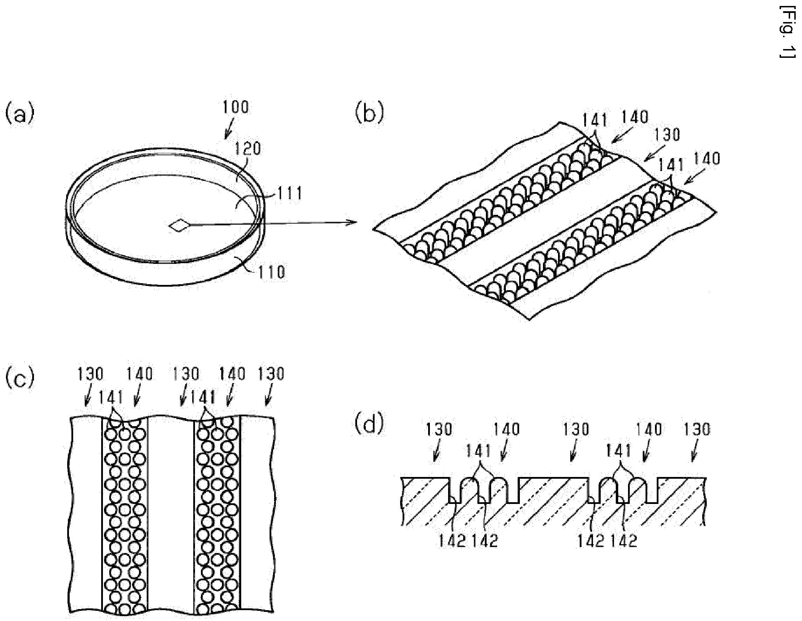

As shown in Fig. 1(a), the alternating arrangement portion 100 is, for example, a sheet material placed on a culture dish 110 of a petri dish. The petri dish retains the cell suspension in a space surrounded by the culture dish 110 and a lid 120. The bottom of the petri dish may be processed into a surface shape having a specific flat portion and a recession and protrusion portion as described above, where the petri dish itself is a cell culture substrate including an alternating arrangement portion.

As shown in Fig. 1(b), the surface 111 of the alternating arrangement portion 100 includes a plurality of flat portions 130 and a plurality of recession and protrusion portions 140. The recession and protrusion portions 140 are composed of a plurality of stepped structures, and the plurality of stepped structures fill a gap between the flat portions 130 adjacent to each other. The stepped structure is a protrusion portion. The recession and protrusion portion 140 includes a recession portion sandwiched between flat portions 130 adjacent to each other and a plurality of protrusion portions 141 located in a bottom surface of the recession portion.

As shown in Fig. 1(c), each flat portion 130 is a flat surface extending in one direction, a first direction (top-bottom direction of Fig. 1(c)). Each flat portion 130 is arrayed in a second direction (left-right direction of Fig. 1(c)) orthogonally intersecting the first direction over an entirety of the surface 111. Each recession and protrusion portion 140 also extends in the first direction and is arrayed in the second direction over an entirety of surface 111.

Each protrusion portion 141 constituting the recession and protrusion portion 140 is located, for example, at each vertex of the triangular grid, seen from a direction opposite the surface 111. Each recession and protrusion portion 140 repeats such an alignment of the protrusion portions 141 in the first direction and the second direction. When the recession and protrusion portion 140 is one in which the protrusion portions 141 are located at each vertex of a triangular grid, an original plate for forming the protrusion portion 141 can be formed by an etching method using a mask suitable for forming a micro-repetitive structure, for example, using a single particle film as a mask.

Seen from the direction opposite the surface 111, each protrusion portion 141 has, for example, a circular shape. The mode of the distance between the centers of the protrusion portions 141 adjacent to each other is a pitch of the protrusion portion 141. The maximum width of the protrusion portion in the planar view shape of the protrusion portion 141 is the diameter of the protrusion portion 141.

The configuration in which the pitch of the protrusion portion 141 satisfies the following (A) and (B) is suitable from the viewpoint of aligning the extension direction of the cardiomyocytes in the first direction. That is, the configuration in which the pitch of the protrusion portion 141 satisfies the following (A) and (B) is suitable from the viewpoint that advantages and disadvantages for adhesion to the cardiomyocyte are clearly divided between the flat portion 130 and the recession and protrusion portion 140.

(A) Pitch of protrusion portion 141: 100 nm or more and 10 $\mu$m or less
(B) Diameter of protrusion portion 141: 50% or more and 100% or less of pitch of protrusion portion 141

The length of each flat portion 130 in the second direction (short side direction) is a width of the flat portion 130. The length in the second direction (short side direction) between flat portions 130 adjacent to each other is a width of the recession and protrusion portion 140.

The width of the flat portion 130 and the width of the recession and protrusion portion 140 are, for example, 1/10 times or more and 10 times or less in the size of the cell (5 $\mu$m or more and 100 $\mu$m or less) to be cultured. The configuration in which the width of the flat portion 130 and the width of the recession and protrusion portion 140 satisfies the following (C) and (D) is suitable from the viewpoint facilitating aligning the extension direction of the cardiomyocyte in the first direction.

(C) Width of flat portion 130: 10 $\mu$m or more and 50 $\mu$m or less
(D) Width of recession and protrusion portion 140: 10 $\mu$m or more and 50 $\mu$m or less

As shown in Fig. 1(d), the recession and protrusion portion 140 may include a recession potion 142 between protrusion portions 141 adjacent to each other and between the flat portion 130 and the adjacent protrusion portion 141. Since a plurality of the protrusion portions 141 are dotted in the recession and protrusion portion 140, the recession portions 142 that are spaces between the protrusion portions 141 are present in a row in the first direction and the second direction in the recession and protrusion portion 140.

In the thickness direction of the alternating arrangement portion 100, the length between the bottom surface of the recession portion 142 and the flat portion 130 is a height of the flat portion 130. In the thickness direction of the alternating arrangement portion 100, the height difference between a distal end surface of each protrusion portion 141 and the flat portion 130 is a boundary step. The height difference between the bottom surface of the recession portion 142 and the distal end surface of each protrusion portion 141 is a height of the protrusion portion 141. In a configuration in which the distal end surface of each protrusion portion 141 and the flat portion 130 are in one surface, the height of the flat portion 130 and the height of the protrusion portion 141 are equal to each other. The ratio of the pitch of the protrusion portion 141 to the height of the protrusion portion 141 is an aspect ratio of the protrusion portion 141.

The configuration in which the boundary step satisfies the following (E) is suitable from the viewpoint of increasing the flatness of the cardiomyocyte sheet. The configuration in which the height of the protrusion portion 141 satisfies the following (F) and the configuration in which the aspect ratio of the protrusion portion 141 satisfies the following (G) are suitable from the viewpoint of increasing the stability in structure of the recession and protrusion portion 140 and the viewpoint of facilitating forming the recession and protrusion portion 140.

(E) Boundary step: 0.5 $\mu$m or less, preferably 0.3 $\mu$m or less
(F) Height of protrusion portion 141: 50 nm or more and 5 $\mu$m or less
(G) Aspect ratio of protrusion portion 141: 0.1 or more and 10 or less

Then, when the configuration satisfies the above (A) and (B), cells in which adhesion to the flat portion 130 is predominant adhere preferentially to the flat portion, and coupled with the inferior adhesion to the recession and protrusion portion 140, the extension directions of the cells align in the first direction that is the extension direction of both structures. As a result, in the cardiomyocyte sheet spreading in two-dimensional direction along the surface 111, it is possible to align the extension direction of the cells in one-dimensional direction, that is, to improve the alignment of the cells.

The configuration that satisfies the above (E), in particular, the configuration in which the distal end surface of each protrusion portion 141 and the flat portion 130 are in one surface, enables the flatness of the cardiomyocyte sheet formed to cover the recession and protrusion portion 140 and the flat portion 130 to increase. Furthermore, the configuration satisfying the above (F) enables the flatness of the cardiomyocyte sheet to further increase.

The surface 111 of the alternating arrangement portion 100 may be a surface in which an organic substance including, for example, an extracellular matrix such as laminin, collagen, gelatin, fibronectin, polylysine (PDL or PLL), or hyaluronic acid, a polymer, or an adhesion factor such as a gel is applied or may be a surface composed of a metal for the purpose of enhancing the adhesion of cells. The surface 111 of the alternating arrangement portion 100 may be hydrophilic or hydrophobic for the purpose of enhancing the adhesion of cells or the flatness of the cell sheet.

In addition, a stimulus-responsive material may be applied to facilitate peeling and recovery of the cell sheet after forming the cell sheet. As the stimulus-responsive material, the temperature-responsive polymer whose water affinity changes depending on a temperature change is preferred. Specifically, it is preferable that the stimulus-responsive material is poly-N-isopropyl acrylamide (PIPAAm). The stimulus-responsive material may be applied to the substrate using a conventional application method, or the structure may be processed into a substrate treated with the stimulus-responsive material using the method described below.

(Method for producing cell culture substrate including alternating arrangement portion)

An example of a method for producing a cell culture substrate including an alternating arrangement portion is described. Note that, in the example described below, a surface 111 of an alternating arrangement portion is formed by transcription of an intaglio 150 using a nano-imprint method.

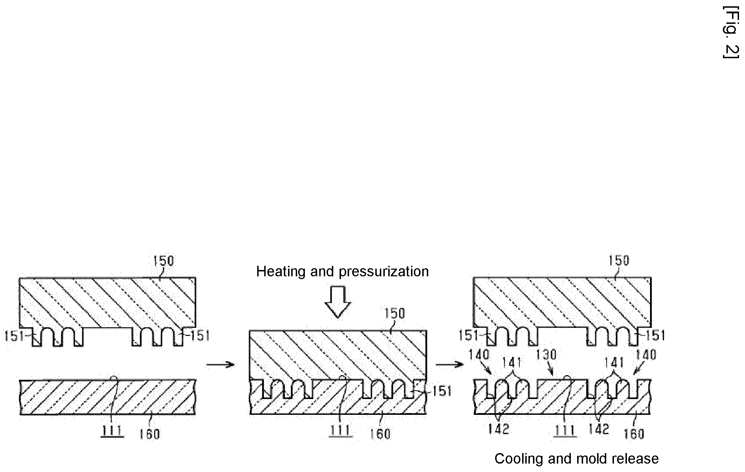

As shown in Fig. 2, the method for producing a cell culture substrate including an alternating arrangement portion includes a step of forming an intaglio 150 and a step of forming a surface 111 of an alternating arrangement portion 100 with transcription of the intaglio 150.

The underside of the intaglio 150 has a shape extending in a first direction (the direction orthogonal to a paper surface) and includes a plurality of flat portions arrayed in a second direction (the left-right direction of the paper surface) intersecting the first direction and a plurality of recession and protrusion portions composed of a stepped structure filling a gap between flat portions adjacent to each other. The flat portion of the intaglio 150 is a portion for forming the flat portion 130 of alternating arrangement portion 100 by transcription. The recession and protrusion portion of the intaglio 150 is a portion for forming the recession and protrusion portion 140 of the alternating arrangement portion 100 by transcription.

The stepped structure of the intaglio 150 is a protrusion portion or a recession portion. It should be noted that the stepped structure of the intaglio 150 in the present embodiment is a recession portion 151 for forming a protrusion portion 141, and the pitch of the recession portion 151 is 100 nm or more and 10 $\mu$m or less. In the step of forming an intaglio 150, a recession and protrusion portion is formed using, for example, at least one of a photolithography method, a colloidal lithography method, an anodic oxidation method, and an interference exposure method for a silicon substrate for forming the intaglio 150. The intaglio 150 itself may be obtained by one or more transcriptions from an original plate. The original plate has a formed shape corresponding to the surface shape of the intaglio 150 using, for example, at least one of a photolithography method, a colloidal lithography method, an anodic oxidation method, and an interference exposure method for a silicon substrate.

The surface 111 of the substrate 160 for forming the alternating arrangement portion 100 is then opposed to the underside of the intaglio 150. The forming material of the substrate 160 is, for example, a thermoplastic resin or a photocurable resin. The underside of the intaglio 150 is then pressed onto the surface 111 of the substrate 160 while the substrate 160 has fluidity. The intaglio 150 is then released from the surface 111 of the substrate 160 while the fluidity of the substrate 160 is suppressed. As a result, a recession portion 151 of the intaglio 150 is transcribed onto the surface 111 of the substrate 160 to form a flat portion 130 and a recession and protrusion portion 140.

To the surface of a thermoplastic resin or photocurable resin, which is a forming material of the substrate 160, an organic substance including, for example, an extracellular matrix such as laminin, collagen, gelatin, fibronectin, polylysine (PDL or PLL),or hyaluronic acid, a polymer, or an adhesion factor such as a gel may be applied for the purpose of enhancing the adhesion of the cells. A biomaterial such as a polysaccharide or a protein may also be used as the forming material of the substrate 160.

<Method for producing cell culture substrate with laminated cardiomyocyte sheets>

A method for producing a cell culture substrate with laminated cardiomyocyte sheets produced using a cell culture substrate including an alternating arrangement portion 100 is described.

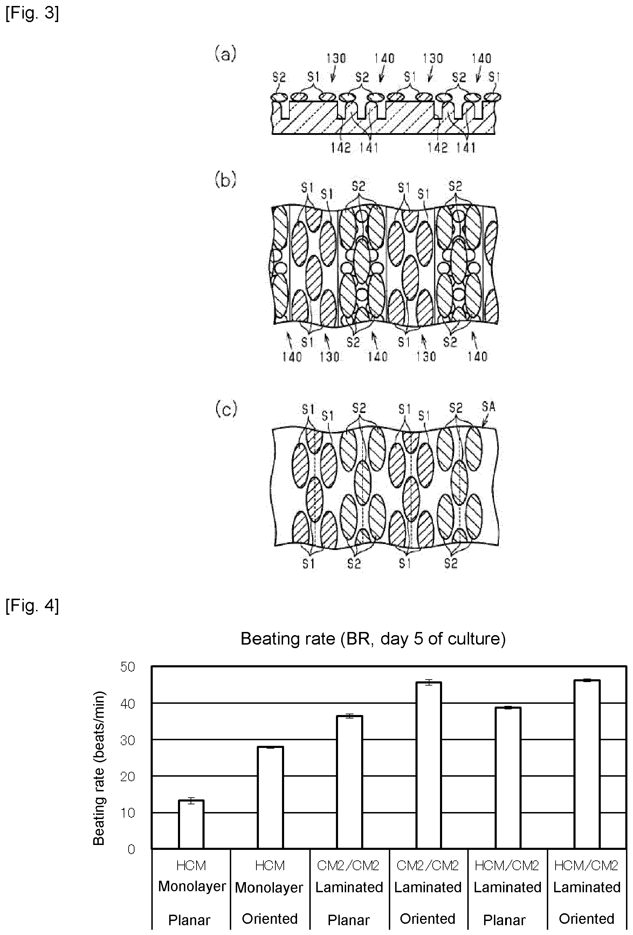

In a cell culture substrate including an alternating arrangement portion satisfying the above (A), as shown in Fig. 3(a), a cell in the cell suspension retained in the alternating arrangement portion 100 may be a cell S1 that preferentially adheres to the flat portion 130 or a cell S2 that is allowed to adhere to the recession and protrusion portion 140 although the adhesion is inferior to that to the flat portion 130.

In this case, as shown in Fig. 3(b), the flat portion 130 and the recession and protrusion portion 140 extend in the first direction and are alternately arranged in the second direction. Thus, in the surface 111 of the alternating arrangement portion, for example, the alignment of the cell S1 that is preferentially adhered to the flat portion 130 is controlled by the structure of the flat portion 130 and the structure of the recession and protrusion portion 140 that compartmentalizes the flat portion 130.

In the recession and protrusion portion 140 sandwiched between the flat portions 130 adjacent to each other, the control of the alignment by the flat portion 130 is reflected in the cell S2 adhered to the recession and protrusion portion 140 although the adhesion is inferior to that to the flat portion 130. As a result, as shown in Fig. 3(c), a cultured cell sheet SA in which the cells S1 and S2 whose alignments are controlled in the first direction are extended across the surface 111 is formed.

Accordingly, the cardiomyocyte sheet on the aligned substrate imparts alignment to the cardiomyocytes.

The cell culture substrate with laminated cardiomyocyte sheets of the present embodiment is preferably produced by any of the following methods 1 to 3.

(Method 1)

A method for producing a cell culture substrate with laminated cardiomyocyte sheets, the method including in order the following steps of:

preparing a cell culture substrate including an alternating arrangement portion in which a strip-shaped cell adhesion region and a strip-shaped cell adhesion suppression region are alternately arranged;
forming a normal cardiomyocyte sheet containing a differentiation-induced cardiomyocyte derived from a healthy human on at least the alternating arrangement portion of the cell culture substrate; and
forming a disease cardiomyocyte sheet containing a differentiation-induced cardiomyocyte derived from a disease patient on the normal cardiomyocyte sheet.

(Method 2)

A method for producing a cell culture substrate with laminated cardiomyocyte sheets, the method including in order the following steps of:

preparing a cell culture substrate including an alternating arrangement portion in which a strip-shaped cell adhesion region and a strip-shaped cell adhesion suppression region are alternately arranged;
forming a disease cardiomyocyte sheet containing a differentiation-induced cardiomyocyte derived from a disease patient on at least the alternating arrangement portion of the cell culture substrate; and
forming a normal cardiomyocyte sheet containing a differentiation-induced cardiomyocyte derived from a healthy human on the disease cardiomyocyte sheet.

(Method 3)

A method for producing a cell culture substrate with laminated cardiomyocyte sheets, the method including the following steps of:

forming a disease cardiomyocyte sheet containing a differentiation-induced cardiomyocyte derived from a disease patient on at least an alternating arrangement portion of a cell culture substrate including the alternating arrangement portion in which a strip-shaped cell adhesion region and a strip-shaped cell adhesion suppression region are alternately arranged;

forming a normal cardiomyocyte sheet containing a differentiation-induced cardiomyocyte derived from a healthy human on at least an alternating arrangement portion of a cell culture substrate including the alternating arrangement portion in which a strip-shaped cell adhesion region and a strip-shaped cell adhesion suppression region are alternately arranged; and

separating one of the disease cardiomyocyte sheet and the normal cardiomyocyte sheet from the cell culture substrate and laminating it on the other cardiomyocyte sheet.

In Method 1, a normal cardiomyocyte sheet containing normal cardiomyocytes is formed on at least an alternating arrangement portion of the cell culture substrate including the alternating arrangement portion described above. That is, the normal cardiomyocytes are seeded and cultured so as to contact the alternating arrangement portion to form the normal cardiomyocyte sheet. It should be noted that the normal cardiomyocyte sheet is required to be formed only on at least the alternating arrangement portion, and the normal cardiomyocyte sheet may be formed on the entire surface of the cell culture substrate. When the normal cardiomyocyte sheet is formed on the alternating arrangement portion, a normal cardiomyocyte sheet in which the cells are aligned in the first direction is formed.

After the normal cardiomyocyte sheet is formed, disease cardiomyocytes are seeded and cultured to form a disease cardiomyocyte sheet on the normal cardiomyocyte sheet. It should be noted that the disease cardiomyocyte sheet is required to be formed only on the normal cardiomyocyte sheet formed on at least the alternating arrangement portion, and the disease cardiomyocyte sheet may be formed on the entire surface of the cell culture substrate. When the disease cardiomyocyte sheet is formed on the aligned normal cardiomyocyte sheet, the disease cardiomyocyte sheet is also aligned in the same manner as the normal cardiomyocyte sheet.

In Method 1, a laminated cardiomyocyte sheet in which the cell culture substrate, the normal cardiomyocyte sheet, and the disease cardiomyocyte sheet are laminated in this order is produced.

In Method 2, a disease cardiomyocyte sheet containing disease cardiomyocytes is formed on at least an alternating arrangement portion of the cell culture substrate including the alternating arrangement portion described above. That is, the disease cardiomyocytes are seeded and cultured so as to contact the alternating arrangement portion to form the disease cardiomyocyte sheet. It should be noted that the disease cardiomyocyte sheet is required to be formed only on at least the alternating arrangement portion, and the disease cardiomyocyte sheet may be formed on the entire surface of the cell culture substrate. When the disease cardiomyocyte sheet is formed on the alternating arrangement portion, a disease cardiomyocyte sheet in which the cells are aligned in the first direction is formed.

After the disease cardiomyocyte sheet is formed, normal cardiomyocytes are seeded and cultured to form a normal cardiomyocyte sheet on the disease cardiomyocyte sheet. It should be noted that the normal cardiomyocyte sheet is required to be formed only on the disease cardiomyocyte sheet formed on at least the alternating arrangement portion, and the normal cardiomyocyte sheet may be formed on the entire surface of the cell culture substrate. When the normal cardiomyocyte sheet is formed on the aligned disease cardiomyocyte sheet, the normal cardiomyocyte sheet is also aligned in the same manner as the disease cardiomyocyte sheet.

In Method 2, a laminated cardiomyocyte sheet in which the cell culture substrate, the disease cardiomyocyte sheet, and the normal cardiomyocyte sheet are laminated in this order is produced.

In Method 3, a disease cardiomyocyte sheet is formed by seeding and culturing disease cardiomyocytes on at least an alternating arrangement portion of a cell culture substrate including the alternating arrangement portion. This forms an aligned disease cardiomyocyte sheet.

Furthermore, a normal cardiomyocyte sheet is formed by seeding and culturing normal cardiomyocytes on at least an alternating arrangement portion of a cell culture substrate including the alternating arrangement portion. This forms an aligned normal cardiomyocyte sheet.

It should be noted that the disease cardiomyocyte sheet and the normal cardiomyocyte sheet are formed on different cell culture substrates.

Then, one of the disease cardiomyocyte sheet and the normal cardiomyocyte sheet is separated from the cell culture substrate and laminated on the other cardiomyocyte sheet. Separation from the cell culture substrate is performed while the cardiomyocytes remain in a sheet form. Examples of a method for this include a method of applying a stimulus-responsive material to the cell culture substrate in advance to facilitate peeling and recovery of the cell sheet. As the stimulus-responsive material, the temperature-responsive polymer whose water affinity changes depending on a temperature change is preferred, and examples thereof include poly-N-isopropyl acrylamide (PIPAAm). The stimulus-responsive material may be applied to the substrate using a conventional application method, or a recession and protrusion structure may be formed in a planar substrate treated with the stimulus-responsive material using the method described above.

It is preferable that the cardiomyocyte sheet separated from the cell culture substrate is laminated so that the alignment direction thereof is the same as the alignment direction of the cardiomyocyte sheet on the cell culture substrate.

When the disease cardiomyocyte sheet is separated from the cell culture substrate and laminated on the normal cardiomyocyte sheet, a cell culture substrate with laminated cardiomyocyte sheets in which the cell culture substrate, the normal cardiomyocyte sheet, and the disease cardiomyocyte sheet are laminated in this order is obtained.

When the normal cardiomyocyte sheet is separated from the cell culture substrate and laminated on the disease cardiomyocyte sheet, a cell culture substrate with laminated cardiomyocyte sheets in which the cell culture substrate, the disease cardiomyocyte sheet, and the normal cardiomyocyte sheet are laminated in this order is obtained.

[Evaluation method of normal cardiomyocyte sheet for biological transplantation]

The cell culture substrate with cardiomyocyte sheets of the present embodiment includes a lamination of a disease cardiomyocyte sheet and a normal cardiomyocyte sheet, and can be used as an evaluation model for the curative effect on a disease when a normal cardiomyocyte sheet is biologically transplanted into the heart of a patient with a cardiac disease.

It is preferable that the evaluation method has a step of evaluating at least one change selected from a change in motor function and a change in physiological property of the disease cardiomyocyte sheet laminated with the normal cardiomyocyte sheet.

Examples of the change in motor function include the change that can be observed by live cell imaging. Examples of the physiological property include state changes such as electrophysiological changes, life and death and changes in shape measured by Ca-imaging analysis, patch clamp method, or the like.

It should be noted that when the motor function and physiological property in the disease cardiomyocyte sheet alone approach the motor function and physiological property in normal cardiomyocytes, it is determined that there is a curative effect. At this time, the motor function and physiological property in the normal cardiomyocyte sheet alone may be employed as a positive control.

<Live cell imaging>

Changes in motion associated with cardiomyocyte contraction and relaxation can be detected with the cardiomyocyte sheet composed of cardiomyocytes. It is observed in vivo that cardiomyocytes are aligned in one direction and contract and relax in the alignment direction.

A beating rate (BR), a contractile velocity (CV), a relaxation velocity (RV), a contraction-relaxation duration (CRD) and a degree of alignment can be analyzed using a live cell imaging device having a microscope and a camera capable of video shooting (e.g., SI8000, manufactured by Sony Corporation) by shooting the cultured cell sheet (live cells) under the culture conditions to obtain a video data, and detecting motion vectors (velocity, direction, quantity) associated with the contraction and relaxation of cardiomyocytes from the video data. For the measurement of BR, CV, RV, CRD, and the like by live cell imaging, see, for example, Methods in Molecular Biology, vol. 2320, Chapter 15.

For the measurement method of these, see also the method described in Examples.

Here, the degree of alignment is calculated by determining the angle of motion vector with regarding the screen horizontal direction of the image obtained by the measurement as 0°, and determining a ratio of the number of vectors showing an angle of $\pm 5°$ from an angle of the mode of the vector angle to the sum of the number of vectors detected. The degree of alignment obtained from the live cell imaging device is determined by the following equation.

Degree of alignment (%) = (number of vectors included in range of $\pm 5°$ of mode)/(total number of vectors) $\times$ 100

In the present embodiment, the degree of alignment measured using the live cell imaging in any layer of the laminated cardiomyocyte sheets is preferably 10% or more, more preferably 12% or more, further preferably 16% or more, even more preferably 18% or more, and particularly preferably 20% or more on the aligned substrate. The upper limit of the degree of alignment is not particularly limited. When the degree of alignment is within the above range, the cardiomyocyte sheet is cultured in a state closer to in vivo, and can be evaluated in an environment closer to in vivo.

In the live cell imaging, the adult beating rate is on average 60 beats/min, thus generally a beating rate (BR) closer to 60 beats/min is closer to normal mature cardiomyocytes. In addition, it is considered that the contractile velocity (CV), the relaxation velocity (RV), and the contraction-relaxation duration (CRD) in accordance with the normal beating rate are closer to normal cardiomyocytes. Normal cardiomyocytes may be cultured in a similar manner, and the measured BR, CV, RV, CRD, and the like may be used as positive controls (target values for normalization).

When the BR, CV, RV, CRD, or the like measured in the disease cardiomyocyte sheet alone changes to a state closer to the normal by the lamination to a normal cardiomyocyte sheet, it is believed that a curative effect on a disease can be obtained when a biological transplantation with the normal cardiomyocyte sheet is performed.

It is also possible to measure the curative effect by the degree of the change.

<Ca-imaging analysis>

In cardiomyocytes, a phenomenon called calcium transient in which concentrations of Ca in the whole cytoplasm provoked by the action potential increase about the same time along with myocardial contraction is observed. The calcium transient can be observed over time by treating the cardiomyocyte sheet of the present embodiment with a fluorescent Ca indicator and observing with a confocal quantitative image cytometer (Ca-imaging analysis). A dedicated analysis software is used as a method to create a waveform graph from the signal information of calcium transient and analyze the parameters described below.

In cardiomyocytes with some abnormalities due to immature cardiomyocytes or a disease, the line width (duration) of 50% height of the waveform peak of calcium transient tends to be extended compared to that of normal mature cardiomyocytes.

Since the line width (duration) of 50% height varies depending on the beating rate, it is preferable to use Duration/(Interval)$^{1/2}$, that is the duration (seconds) divided by the 1/2 power of interval (seconds).

In general, it tends to be closer to mature cardiomyocytes as the line width (duration) of 50% height that reflects the calcium transient duration is shorter. It can be said that when the line width of 50% height is shortened, the disease cardiomyocytes become closer to normal cardiomyocytes. The interval is affected by the heartbeat. In accordance with the analysis of electrocardiogram in which the value of action potential duration corrected with the beating rate is usually employed, Duration/(Interval)$^{1/2}$ as described above may be employed.

Thus, when the duration of calcium transient obtained in the disease cardiomyocyte sheet becomes equivalent to that of normal cardiomyocytes by the lamination of the normal cardiomyocyte sheet, it is believed that a curative effect can be obtained by transplantation with the normal cardiomyocyte sheet.

<Patch clamp method>

In the patch-clamp test, a current of 20 pA to 100 pA is applied at 0.2 Hz under a current clamp into cardiomyocytes in a Tyrode solution at 25°C to generate an action potential, and the change in potential is measured. The current can be selected as appropriate to the extent that it can generate an appropriate action potential.

Examples of the physiological property include a maximum diastolic potential and an action potential duration.

Examples of an indicator of the action potential duration include an action potential duration at 80% repolarization. The action potential duration at 80% repolarization is the time from the application of current to the time required to become a potential equivalent to 80% repolarization when the difference between the peak height of the action potential shown as the first phase and the maximum diastolic potential is 100%. That is, it is the time from the rise time of action potential to the time required for the action potential to become a potential equivalent to 80% repolarization.

When cardiomyocytes mature, the action potential duration tends to be longer and the maximum diastolic potential tends to be deeper compared to immature cardiomyocytes.

In the present embodiment, the action potential duration at 80% repolarization of the disease cardiomyocytes of the laminated cardiomyocyte sheets is preferably 600 msec or more, more preferably 650 msec or more, further preferably 700 msec or more, and even more preferably 750 msec or more, and the upper limit is not particularly limited, but it is preferably 1200 msec or less, more preferably 1050 msec or less, and further preferably 900 msec or less from the viewpoint that it is preferable that the action potential duration is about the same as that of cardiomyocytes in vivo.

In addition, the maximum diastolic potential of the diseased cardiomyocytes constituting the cell culture substrate with laminated cell sheets of the present embodiment is preferably -60 mV or less, more preferably -62.5 mV or less, further preferably -65 mV or less, and even more preferably -67.5 mV or less from the viewpoint that it is preferable that the maximum diastolic potential value thereof is closer to that of cardiomyocytes in vivo. Although the lower limit is not particularly limited, it is preferably a value close to -80 mV since the value of the adult ventricular cardiomyocytes is about -80 mV.

For the action potential duration and the maximum diastolic potential (resting membrane potential) in human-derived normal isolated ventricular myocytes, see Circulation, 2013; 127: 575-584.

<Others>

As at least one change selected from a change in motor function and a change in physiological property of the disease cardiomyocyte sheet, a cell-killing effect on the disease cardiomyocyte sheet may be evaluated. No cell-killing effect on the disease cardiomyocyte sheet by the lamination of the normal cardiomyocyte sheet may be evaluated by means of trypan blue staining or the like.

The shape change of the diseased cardiomyocytes in the diseased cardiomyocyte sheet may also be evaluated.

<Application of cell culture substrate with laminated cardiomyocyte sheets>

The cell culture substrate with laminated cardiomyocyte sheets of the present embodiment can be used for various applications, for example, for evaluation of a curative effect in regenerative medicine. It can be evaluated to what extent the motor function and the physiological property change closer to those of the normal cardiomyocyte sheet by laminating the normal cardiomyocyte sheet to the disease cardiomyocyte sheet. It can also be evaluated whether changes in shape of cardiomyocytes constituting the disease cardiomyocyte sheet occur or whether decreases in the number of viable cells do not occur by the lamination of the normal cardiomyocyte sheet.

Specifically, the cell culture substrate with laminated cardiomyocyte sheets of the present embodiment can be used to evaluate a curative effect in a case of transplanting a normal cardiomyocyte sheet to a damaged site in the myocardial tissue of a heart damaged by an ischemic disease such as myocardial infarction or angina pectoris (hereinafter, also referred to as "damaged heart") in the same direction as the contraction of the cardiac tissue by a method of directly attachment, suturing after attachment, insertion or the like.

When the normal cardiomyocyte sheet is used in actual regenerative medicine, a single layer of normal cardiomyocyte sheet may be used or several multilayer normal cardiomyocyte sheets may be used after being overlaid and laminated. In the lamination, it is preferable that the alignment directions are aligned. Specifically, the lamination may be performed while aligning the contraction directions or alignment directions of the normal cardiomyocyte sheets.

Examples

Hereinafter, Examples are given to specifically illustrate the present invention, but the present invention is not limited to these Examples.

In Examples, hypertrophic cardiomyopathy cardiomyocytes (HCM) derived from iPS cells were used as a model for a cardiac disease, and cardiomyocytes (CM2) derived from iPS cells were used as normal cardiomyocytes. First, in order to reproduce the environment of the heart in vivo, normal cardiomyocytes were seeded and cultured on an aligned substrate to produce a normal cardiomyocyte sheet in which the longitudinal directions of cardiomyocytes were aligned in one direction. After the normal cardiomyocyte sheet in which cells were aligned was formed, hypertrophic cardiomyopathy cardiomyocytes (HCM) were seeded onto the normal cardiomyocyte sheet to form a hypertrophic cardiomyopathy cardiomyocyte sheet on the normal cardiomyocyte sheet. The sheet was subjected to a maintenance culture for a predetermined number of days, and then the function of the hypertrophic cardiomyopathy cardiomyocyte sheet was measured, and the therapeutic effect by transplantation was evaluated.

As controls, a cell culture substrate with a disease cardiomyocyte sheet in which HCM was seeded and cultured in monolayer on a planar substrate or an aligned substrate, and a cell culture substrate with laminated normal cardiomyocyte sheets in which CM2 was seeded and cultured in lamination on a planar substrate or an aligned substrate were prepared and evaluated in the same manner.

It should be noted that the results obtained using the cell culture substrate with a monolayer normal cardiomyocyte sheet prepared by seeding and culturing CM2 in monolayer were equivalent to the results obtained using the cell culture substrate with laminated normal cardiomyocyte sheets prepared by laminating CM2 and CM2.

(1) Culture of human iPS cell-derived cardiomyocytes (normal cardiomyocytes) (CM2)

iCell cardiomyocytes (iCell Cardiomyocyte v2.0, manufactured by FUJIFILM Cellular Dynamics) were used as human iPS cell-derived cardiomyocytes (hereinafter referred to as CM2). The culture was performed by the following procedure.

Frozen cell samples were warmed in a 37°C water bath for 3 minutes and lysed. The cell solution was transferred to a 50 mL centrifuge tube, and 9 mL of plating medium (iCell cardiomyocyte thawing medium, manufactured by FUJIFILM Cellular Dynamics, Inc.) prewarmed to 37°C was added and mixed to dilute the cell solution.

A portion of the diluted cell suspension was collected, mixed with an equal amount of trypan blue, and the viable cells were counted using a hemocytometer.

The cell concentration was adjusted using a plating medium and the cells were seeded at a concentration of 6 ×

$10^4$ cells/well into a 96-well plate coated in advance with fibronectin. The 96-well plates into which the cells were seeded were an aligned substrate (aligned plate, ND Cell Aligner, 96-well plate type) and a commercially available 96-well plate having a flat culture surface (planar plate) as a control.

The cells after seeding were allowed to stand for 4 hours in a 37°C $CO_2$ incubator, and the medium was exchanged using a maintenance medium (iCell cardiomyocyte maintenance medium, manufactured by FUJIFILM Cellular Dynamics). The cells after the medium exchange were subjected to the medium exchange every 2 days, followed by the maintenance culture for a predetermined period.

The ND Cell Aligner used in (1) above and Examples described below had strip-shaped flat portions (cell adhesion regions) and strip-shaped recession and protrusion portions (cell adhesion suppression regions), in which each flat portion had a shape extending in a first direction and the flat portions were arrayed in a second direction intersecting the first direction over an entirety of the surface, and the width (length in the second direction) of each flat portion was 10 $\mu$m. Each recession and protrusion portion was composed of a plurality of stepped structures filling a gap between flat portions adjacent to each other, the length between respective flat portions in the second direction was 10 $\mu$m, and the pitch of the protrusion portion in the recession and protrusion portion was 300 nm. As a result of measuring the height of each protrusion portion in the recession and protrusion portion using AFM, the average height from the bottom surface of the recession portion to the distal end of the protrusion portion was 446 nm. The average height of the flat portion from the bottom surface of the recession portion was 455 nm.

(2) Culture of diseased human iPS cell-derived cardiomyocytes (disease cardiomyocytes) (HCM)

Cardiomyocytes derived from diseased human iPS cells (Mycell cardiomyocytes (MYH7 R403Q) 01178 (FUJIFILM Cellular Dynamics, Inc.))(hereinafter referred to as HCM) were used as cardiomyocytes indicating the pathology of hypertrophic cardiomyopathy. The cells are disease cardiomyocytes of hypertrophic cardiomyopathy in which cardiomyocytes have been differentiated from iPS cells of a disease patient having a missense mutation (R403Q) in which arginine at 403 residue of the β-myosin heavy chain 7 gene is mutated to glutamine. The culture was performed by the same procedure as in CM2.

The viable cells in the cell solution were counted, and the cell concentration of the cell solution was adjusted using a plating medium, and then the cells were seeded at a concentration of $6 \times 10^4$ cells/well into a 96-well plate culture vessel.

(3) Implementation of monolayer culture

HCM was cultured on a planar plate and an aligned plate, and the motor function of the cell sheets cultured on each culture substrate was measured and evaluated.

In accordance with the procedure described in (2) above, HCM was seeded on a planar plate and an aligned plate at a concentration of $6 \times 10^4$ cells/well and cultured.

The day of seeding was set as day 0, and various parameters were measured on day 5 of culture with a live cell imaging device (SI8000, manufactured by Sony Corporation). Furthermore, various parameters were measured on day 21 with a confocal fluorescence microscope (confocal quantitative image cytometer CQ1, manufactured by Yokogawa Electric Corporation).

(4) Implementation of lamination culture

The lamination culture was performed by initially seeding and culturing cells (CM2) that form a lower layer with setting the day of seeding as day 0, and then on day 10 seeding and culturing cells (CM2 or HCM) that form an upper layer on the cells of the lower layer.

In accordance with the procedure described in (1) above, CM2 that form the lower layer was seeded and cultured on a planar plate and an aligned plate at a concentration of $6 \times 10^4$ cells/well. On day 10, CM2 or HCM as the upper layer was seeded and cultured at a concentration of $6 \times 10^4$ cells/well on CM2 in culture. With setting the starting day of the lamination culture as day 0, image data was acquired on day 5 with a live cell imaging device (SI8000, manufactured by Sony Corporation). Image data was also acquired on day 21 with a confocal fluorescence microscope (confocal quantitative image cytometer CQ1, manufactured by Yokogawa Electric Corporation).

(5) Maturation confirmation test: analysis by motion test method using live cell imaging device

To detect motion changes associated with the contraction and relaxation of cardiomyocytes seeded on each culture plate, video data was obtained by a live cell imaging device (SI8000, manufactured by Sony Corporation). The measurement was performed under conditions of phase contrast image, magnification of 10× (objective lens), 150 frames/sec, resolution 2048 × 2048 pixels, and 8-bit depth. The image acquisition time was 10 seconds.

From the video data, motion vectors (velocity, direction, quantity) associated with the contraction and relaxation of cardiomyocytes were detected, and each parameter of a beating rate (BR), a contractile velocity (CV), a relaxation velocity (RV), a contraction-relaxation duration (CRD) and a degree of alignment was analyzed. The contraction-relaxation duration (CRD) was corrected by the following equation with reference to the equation of Frederica to correct variations due to beating rate (BR).

$$CRD \text{ (correction value)} = CRD/(60/BR) \times x \ (1/3)$$

The degree of alignment was calculated based on the following equation by determining the angle of motion vector with setting the horizontal direction of the screen as 0°, and determining a ratio of the number of vectors indicating an angle of $\pm 5°$ from an angle of the mode of the vector angle to the sum of the number of vectors detected.

Degree of alignment (%) = (number of vectors included in range of $\pm 5°$ of mode)/(total number of vectors) $\times$ 100

It should be noted that generally there are tendencies that the higher the beating rate (BR), the closer to mature cardiomyocytes; the faster the contractile velocity (CV) and the relaxation velocity (RV), the closer to mature cardiomyocytes; and the shorter the contraction-relaxation duration (CRD), the closer to mature cardiomyocytes.

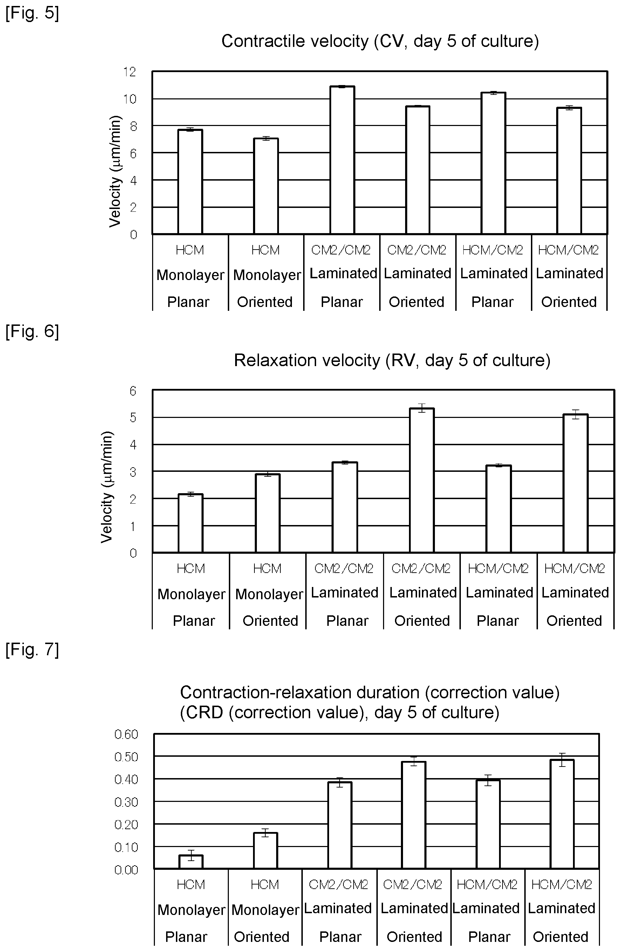

Each measurement result using the live cell imaging device is shown in Figs. 4 to 8.

(6) Maturation confirmation test: Ca-imaging analysis using confocal imaging device

The change in Ca fluorescence intensity associated with the contraction of cardiomyocytes seeded in each culture plate was measured by CQ1 (confocal fluorescence microscope, manufactured by Yokogawa Electric Corporation).

To visualize Ca ion flux, a fluorescent Ca indicator (EarlyTox carditotoxicity kit, manufactured by Molecular Devices, LLC.) was added to a cardiomyocyte sheet and allowed to stand at 37°C for 15 minutes. The measurement of CQ1 was performed under conditions of the excitation wavelength of 488 nm/the fluorescence wavelength of 525 nm and the data acquisition time of 60 seconds. For the analysis, CellPathfinder was used.

From the measurement data, the line width (duration) of 50% height of Ca transient waveform peak and the interval of the waveform peak were selected as parameters and analyzed. The line width (duration) of 50% height of Ca transient waveform peak was corrected by employing Duration/(Interval)$^{1/2}$ to correct variation due to the interval of the waveform peak in accordance with the analysis of electrocardiogram in which the value of the action potential duration corrected with the beating rate is usually employed.

$$Duration \text{ (correction value)} = Duration/(Interval)^{1/2}$$

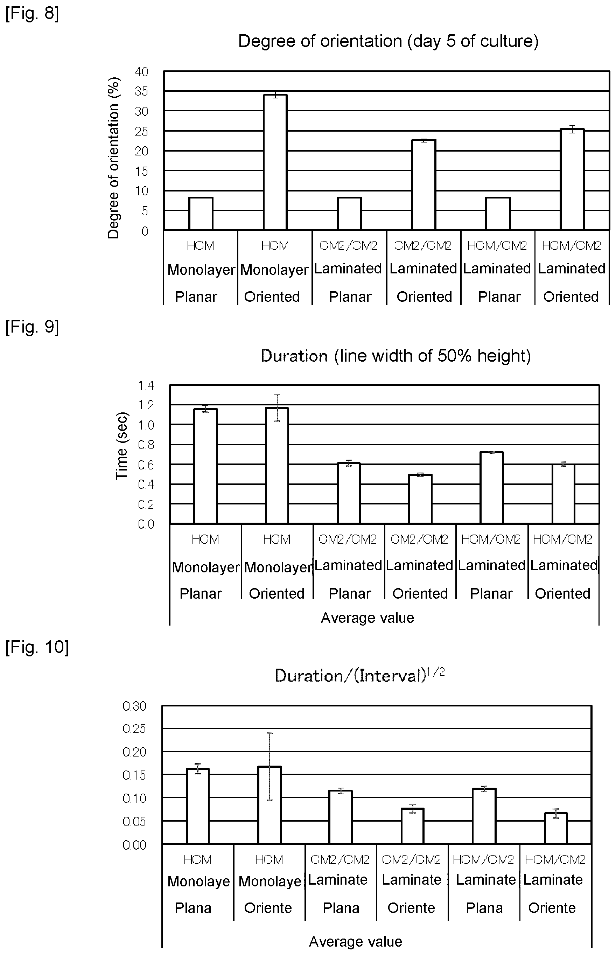

Each measurement result using a confocal imaging device is shown in Figs. 9 and 10.

<Results of lamination culture of iPS-derived cardiomyocytes (CM2) on aligned plate>

The lamination culture was performed by initially seeding and culturing cells (CM2) that form a lower layer. With setting the day of seeding as day 0, cells (CM2) that form an upper layer were seeded on day 10 and cultured on CM2 of the lower layer. With setting the seeding day of the cells of the upper layer as day 0, image data was acquired on day 5 with a live cell imaging device (SI8000, manufactured by Sony Corporation). Image data was also acquired on day 21 with CQ1 (confocal fluorescence microscope, manufactured by Yokogawa Electric Corporation).

From the measurement results by motion vector analysis of SI8000, the lamination-cultured CM2 on the aligned plate showed a significant increase in the beating rate (BR), the relaxation velocity (RV), and the contraction-relaxation duration (CRD, corrected with the beating rate) (all $p < 0.01$) and had significantly high degree of alignment of cells (mode $\pm 5°$) compared to the lamination-cultured CM2 on the planar plate ($p < 0.01$) on day 5 of culture. On the other hand, the lamination-cultured CM2 on the aligned plate had a significantly reduced contractile velocity (CV) ($p < 0.01$) compared to the lamination-cultured CM2 on the planar plate (comparison of planar CM2/CM2 and aligned CM2/CM2 in each graph (Figs. 4 to 8)).

In the measurements with SI8000, data was acquired with focusing on cells in the top layer of the cultured cells. Thus, the measurement values show the results of observing the cells in the upper layer of the lamination culture.

From the measurement results with CQ1, the lamination-cultured CM2 on the aligned plate had a significantly shorter line width of 50% height of the Ca ion transient peak compared to the lamination-cultured CM2 on the planar plate.

In addition, the line width (duration)(correction value) of 50% height of the waveform peak corrected with the interval of the waveform peak was significantly shortened ($p < 0.05$) in the lamination-cultured CM2 on the aligned plate compared to the lamination-cultured CM2 on the planar plate.

In the measurements with CQ1, data was acquired with focusing on cells in the top layer of the cultured cells. Thus, the measurement values show the results of observing the cells in the upper layer of the lamination culture.

In the lamination culture of CM2/CM2 cultured on the aligned plate, normal cardiomyocytes are cultured in a state aligned in one direction as in vivo, and thus they are considered to be in a state close to cardiomyocytes in vivo.

When disease cells are treated by some methods and the symptoms are improved, the measurement result of the disease cells is considered to be close to the numerical values of the lamination-cultured CM2/CM2 cultured on an aligned plate. In the present Example, when each measurement value of HCM becomes closer to the value of CM2, it indicates an improvement of the disease state of HCM.

<Results of monolayer culture of disease human iPS cell-derived cardiomyocytes (HCM) on aligned plate>

From the measurement results by motion vector analysis of SI8000, the monolayer-cultured HCM on the aligned plate showed a significant increase in the beating rate (BR), the relaxation velocity (RV), and the contraction-relaxation duration (CRD, corrected with the beating rate) and had significantly high degree of alignment of cells (mode $\pm5°$) compared to HCM cultured in the planar plate (all $p < 0.01$) on day 5 of culture. On the other hand, the monolayer-cultured HCM on the aligned plate had a reduced contractile velocity (CV) compared to the HCM cultured on the planar plate ($p < 0.01$) (comparison of planar HCM and aligned HCM in each graph (Figs. 4 to 8))).

From the measurement results with CQ1 on day 21 of culture, the monolayer-cultured HCM on the aligned plate had about the same line width of 50% height of Ca ion transient peak compared to the monolayer-cultured HCM on the planar plate.

In addition, the line width (duration)(correction value) of 50% height of the waveform peak corrected with the interval of the waveform peak was about the same between the monolayer-cultured HCM on the aligned plate and the monolayer-cultured HCM on the planar plate.

From the above results, it was confirmed that the beating rate was improved and the relaxation velocity that affects arrhythmia was improved as a result of culturing cardiac disease cells HCM with the aligned plate. This showed that the culture using an aligned plate to culture cells in an aligned state is a culture method that improves arrhythmia of hypertrophic cardiomyopathy cardiomyocytes.

However, the measurement values of the monolayer-cultured HCM on the aligned plate did not reach the measurement values of the lamination-cultured CM2 on the alignment plate in all parameters except for the degree of alignment. It is thus believed that a combination of the aligned culture and another treatment method is needed for further improvement of the symptoms of HCM.

<Results of lamination culture of HCM on normal cardiomyocytes CM2 on planar plate and aligned plate>

The lamination culture was performed by initially seeding and culturing cells (CM2) that form a lower layer. With setting the day of seeding as day 0, cells (HCM) that form an upper layer were seeded and cultured on CM2 of the lower layer on day 10. With setting the seeding day of the cells of the upper layer as day 0, image data was acquired on day 5 with SI8000. Furthermore, image data was acquired on day 21 with CQ1.

From the measurement results by motion vector analysis of SI8000, the lamination-cultured HCM overlaid on CM2 on the aligned plate showed a significant increase in the beating rate (BR), the relaxation velocity (RV), and the contraction-relaxation duration (CRD, corrected with the beating rate) and had significantly high degree of alignment of cells (mode $\pm5°$) on day 5 of culture compared to the lamination-cultured HCM overlaid on CM2 on the planar plate.

On the other hand, the lamination-cultured HCM overlaid on CM2 on the aligned plate had a reduced contractile velocity (CV) compared to the lamination-cultured HCM overlaid on CM2 on the planar plate.

From the measurement results with CQ1, the lamination-cultured HCM overlaid on CM2 on the aligned plate had a significantly shorter line width of 50% height of the Ca ion transient peak compared to the lamination-cultured HCM overlaid on CM2 in the planar plate.

In addition, the line width (duration)(correction value) of 50% height of the waveform peak corrected with the interval of the waveform peak was significantly shortened in the lamination-cultured HCM overlaid on CM2 on the aligned plate compared to the lamination-cultured HCM overlaid on CM2 on the in the planar plate.

From the above results, it was confirmed that the beating rate was improved and the relaxation velocity and the contraction-relaxation duration that affect arrhythmia were improved as a result of lamination culturing cardiac disease cells HCM overlaid on CM2 on the aligned plate. This showed that the culture using an aligned plate to lamination culture cardiac disease cells HCM overlaid on CM2 is a culture method that improves arrhythmia of hypertrophic cardiomyopathy cardiomyocytes.

From the results above, it has been clarified that the culture method of lamination culturing of normal cardiomyocytes and cardiac disease cardiomyocytes in an aligned plate can be used as a model system for cardiomyocyte sheet transplantation. It is possible to evaluate whether cardiomyocytes from a cardiac disease patient can be improved in function by transplanting aligned normal cardiomyocytes or not by culturing cardiomyocytes from a cardiac disease patient overlaid on top of the lower layer of normal cardiomyocytes cultured using an alignment plate, measuring motor function and physiological activity, and analyzing parameters.

Furthermore, it has been suggested that the aligned plate can be used as a tool to produce a cardiomyocyte sheet that performs aligned action for a treatment of heart failure, transplant the cardiomyocyte sheet, and treat arrhythmias.

Reference Signs List

100 Alternating arrangement portion
111 Surface
110 Culture dish
120 Lid
130 Flat portion
140 Recession and protrusion portion
141 Protrusion portion
142 Recession portion

**Claims**

1. A cell culture substrate with laminated cardiomyocyte sheets, comprising two or more layers of cardiomyocyte sheets on a cell culture substrate, wherein

   the cell culture substrate comprises an alternating arrangement portion in which a strip-shaped cell adhesion region and a strip-shaped cell adhesion suppression region are alternately arranged,
   the cell culture substrate with laminated cardiomyocyte sheets comprises the two or more layers of cardiomyocyte sheets on at least the alternating arrangement portion of the cell culture substrate; and
   the cell culture substrate with laminated cardiomyocyte sheets comprises, as the cardiomyocyte sheets, at least one layer of each of a disease cardiomyocyte sheet containing a differentiation-induced cardiomyocyte derived from a disease patient and a normal cardiomyocyte sheet containing a differentiation-induced cardiomyocyte derived from a healthy human.

2. The cell culture substrate with laminated cardiomyocyte sheets according to claim 1, wherein the normal cardiomyocyte sheet is attached on the cell culture substrate and the disease cardiomyocyte sheet is laminated on the normal cardiomyocyte sheet.

3. The cell culture substrate with laminated cardiomyocyte sheets according to claim 1, wherein the disease cardiomyocyte sheet is attached on the cell culture substrate and the normal cardiomyocyte sheet is laminated on the disease cardiomyocyte sheet.

4. A method for producing a cell culture substrate with laminated cardiomyocyte sheets, the method comprising in order the following steps of:

   preparing a cell culture substrate comprising an alternating arrangement portion in which a strip-shaped cell adhesion region and a strip-shaped cell adhesion suppression region are alternately arranged;
   forming a normal cardiomyocyte sheet containing a differentiation-induced cardiomyocyte derived from a healthy human on at least the alternating arrangement portion of the cell culture substrate; and
   forming a disease cardiomyocyte sheet containing a differentiation-induced cardiomyocyte derived from a disease patient on the normal cardiomyocyte sheet.

5. A method for producing a cell culture substrate with laminated cardiomyocyte sheets, the method comprising in order the following steps of:

preparing a cell culture substrate comprising an alternating arrangement portion in which a strip-shaped cell adhesion region and a strip-shaped cell adhesion suppression region are alternately arranged;

forming a disease cardiomyocyte sheet containing a differentiation-induced cardiomyocyte derived from a disease patient on at least the alternating arrangement portion of the cell culture substrate; and

forming a normal cardiomyocyte sheet containing a differentiation-induced cardiomyocyte derived from a healthy human on the disease cardiomyocyte sheet.

6. A method for producing a cell culture substrate with laminated cardiomyocyte sheets, the method comprising the following steps of:

forming a disease cardiomyocyte sheet containing a differentiation-induced cardiomyocyte derived from a disease patient on at least an alternating arrangement portion of a cell culture substrate comprising the alternating arrangement portion in which a strip-shaped cell adhesion region and a strip-shaped cell adhesion suppression region are alternately arranged;

forming a normal cardiomyocyte sheet containing a differentiation-induced cardiomyocyte derived from a healthy human on at least an alternating arrangement portion of a cell culture substrate comprising the alternating arrangement portion in which a strip-shaped cell adhesion region and a strip-shaped cell adhesion suppression region are alternately arranged; and

separating one of the disease cardiomyocyte sheet and the normal cardiomyocyte sheet from the cell culture substrate and laminating it on the other cardiomyocyte sheet.

7. A method for evaluating a normal cardiomyocyte sheet for biological transplantation, the method comprising a step of evaluating at least one change selected from a change in motor function and a change in physiological property of the disease cardiomyocyte sheet of the cell culture substrate with laminated cardiomyocyte sheets according to any one of claims 1 to 3.

[Fig. 1]

[Fig. 2]

Heating and pressurization

Cooling and mold release

[Fig. 3]

[Fig. 4]

Beating rate (BR, day 5 of culture)

[Fig. 5]

**Contractile velocity (CV, day 5 of culture)**

[Fig. 6]

**Relaxation velocity (RV, day 5 of culture)**

[Fig. 7]

**Contraction-relaxation duration (correction value)**
**(CRD (correction value), day 5 of culture)**

[Fig. 8]

**Degree of orientation (day 5 of culture)**

[Fig. 9]

**Duration (line width of 50% height)**

[Fig. 10]

**Duration/(Interval)$^{1/2}$**

## INTERNATIONAL SEARCH REPORT

| International application No. |
| --- |
| **PCT/JP2023/033802** |

| A. | CLASSIFICATION OF SUBJECT MATTER |
| --- | --- |

*C12M 3/00*(2006.01)i; *C12N 5/077*(2010.01)i; *C12Q 1/02*(2006.01)i
FI:   C12M3/00 A; C12N5/077; C12Q1/02

According to International Patent Classification (IPC) or to both national classification and IPC

| B. | FIELDS SEARCHED |
| --- | --- |

Minimum documentation searched (classification system followed by classification symbols)

C12M3/00; C12N5/077; C12Q1/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2013/151755 A1 (UNIVERSITY OF WASHINGTON THROUGH ITS CENTER FOR COMMERCIALIZATION) 10 October 2013 (2013-10-10) claims, paragraphs [0048], [0059], [0100], [0473], fig. 35B, 35C | 1-7 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 November 2023** | **14 November 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/033802**

| Patent document cited in search report | Publication date (day/month/year) | Patent family member(s) | Publication date (day/month/year) |
|---|---|---|---|
| WO 2013/151755 A1 | 10 October 2013 | US 2015/0125952 A1 claims, paragraphs [0048], [0059], [0100], [0656], fig. 35B, 35C | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003306434 A

**Non-patent literature cited in the description**

- Methods in Molecular Biology, vol. 2320
- *Circulation*, 2013, vol. 127, 575-584